(19) Europäisches Patentamt
European Patent Office
Office européen des brevets

(11) **EP 0 858 287 B1**

(12) **EUROPÄISCHE PATENTSCHRIFT**

(45) Veröffentlichungstag und Bekanntmachung des Hinweises auf die Patenterteilung:
**20.07.2005 Patentblatt 2005/29**

(21) Anmeldenummer: **96934709.5**

(22) Anmeldetag: **16.10.1996**

(51) Int Cl.$^7$: **A61B 5/0205**

(86) Internationale Anmeldenummer:
**PCT/EP1996/004497**

(87) Internationale Veröffentlichungsnummer:
**WO 1997/014354 (24.04.1997 Gazette 1997/18)**

(54) **VORRICHTUNG ZUR QUANTITATIVEN ANALYSE VON SCHLAFSTÖRUNGEN**

DEVICE FOR THE QUANTITATIVE ANALYSIS OF SLEEP DISTURBANCES

DISPOSITIF POUR L'ANALYSE QUANTITATIVE DE TROUBLES DU SOMMEIL

(84) Benannte Vertragsstaaten:
**AT BE CH DE DK ES FI FR GB GR IE IT LI LU MC NL PT SE**

(30) Priorität: **16.10.1995 DE 19538473**

(43) Veröffentlichungstag der Anmeldung:
**19.08.1998 Patentblatt 1998/34**

(73) Patentinhaber: **MAP Medizin-Technologie GmbH**
**82152 Martinsried (DE)**

(72) Erfinder: **GRIEBEL, Peter**
**D-86926 Pflaumdorf (DE)**

(74) Vertreter: **VOSSIUS & PARTNER**
**Siebertstrasse 4**
**81675 München (DE)**

(56) Entgegenhaltungen:
| | |
|---|---|
| **EP-A- 0 450 341** | **WO-A-88/02237** |
| **WO-A-91/09372** | **DE-A- 3 533 912** |
| **DE-A- 4 138 702** | **DE-U- 9 204 190** |
| **US-A- 4 802 485** | **US-A- 5 033 472** |
| **US-A- 5 105 354** | **US-A- 5 245 995** |
| **US-A- 5 348 008** | **US-A- 5 385 144** |

## Beschreibung

Die Erfindung betrifft eine Vorrichtung zur quantitativen Analyse von Schlafstörungen, insbesondere zur stationären und ambulanten Aufnahme, Aufzeichnung und Analyse von schlafbezogenen Atmungsstörungen, Herzrhythmusstörungen, Myoklonien, Blutdruckschwankungen, Schlaftiefeparametern, Bewegungsparametern und Störparametern.

Eine stetige Zunahme der Zahl von Personen, die unter Schlafstörungen leiden und die damit verbundene Überlastung von Schlaflaboratorien, die diese Störungen behandeln, hat dazu geführt, die Untersuchung bzw. Messung von Störungen mittels ambulanter Geräte aus dem stationären in den ambulanten Bereich hin zu verlagern. Die Patienten erhalten Anweisungen darüber, wie die mit diesen Geräten verbundenen Sensoren zu behandeln sind und liefern das mobile Aufzeichnungsgerät nach der Meßzeit wieder beim behandelnden Arzt ab, der daraufhin die aufgezeichneten Meßsignale analysiert, um anschließend zu diagnostizieren. Diese ambulante Methode ist aufgrund des mit einer Messung verbundenen hohen Zeitaufwands sehr kostengünstig und außerdem werden die Ergebnisse nicht dadurch beeinflußt, daß die Messungen in einer für den Patienten ungewohnten Umgebung durchgeführt werden.

Ein solcher ambulanter Recorder wird in der EP-A-0 356 603 beschrieben. Dieser Recorder ermöglicht, über acht Kanäle Meßsignale aufzunehmen, diese zu speichern und anschließend in einem Computer zu analysieren. Dabei ist es möglich, den Recorder unterschiedlich zu programmieren, um somit durch eine Vorauswahl die Anzahl der gemessenen Daten zu reduzieren und eine Beschränkung auf gewünschte Meßdaten je nach Krankheitsbild oder Diagnosezweck zu erreichen. Durch diese unterschiedliche Programmierung läßt sich die Messung auf verschiedene Rahmenbedingungen anpassen.

Die Messung verschiedener Parameter zur Erkennung von Schlafstörungen wird in der DE-A-41 38 702 erläutert. Die darin offenbarte Vorrichtung weist Sensoren zur Erfassung des Herzpotentials, der Atmungs- bzw. Schnarchlaute, des Sauerstoffsättigungsgrades des Blutes und der Positionen des Körpers des Patienten auf. Eine Analyse der Meßergebnisse erlaubt die Diagnose von Apnoe. Dieses Analysegerät ist allerdings auf einen einzigen Anwendungsfall, nämlich die Erkennung von Schlafstörungen (Apnoe) beschränkt und besitzt mit den erwähnten Sensoren ein nur auf diesen Zweck zugeschnittenes Instrumentarium zur Analyse. Insbesondere lassen sich zwar schlafbezogene Atmungsstörungen erkennen, jedoch nicht damit verbundene "Nebenwirkungen", wie etwa Herzrhythmusstörungen.

Zum allgemeinen Stand der Technik wird ferner verwiesen auf DE 92 04 190 U1, DE 32 48 222 A1, DE 39 21 784 A1, US 3 734 086, DE 92 00 249 U1 und Kurz, Roland: "Medizinische Meßtechnik und Biosignalverarbeitung in der kardiologischen Diagnostik", M.-G.-Schmitz-Verlag Gießen 1984, S. 122 - 129, ISBN 3-922 272-23-1.

Aus der US-5,348,008 ist ein kardiologisch-respiratorisches Warnsystem bekannt. Der Patient ist mit einem Überwachungssystem verbunden, das Warnsystem kann einen Alarm entsprechend einer vorliegenden Alarmbedingung aufrecht erhalten, und es werden die Bedingungen, die in den Signalen eines Parameters gefunden werden mit den Signalen anderer Parameter verglichen, um die Bedeutung der Signale zu beurteilen, so dass ein Alarm nur ausgelöst wird, wenn lebensbedrohliche Bedingungen bei einem Pattienten festgestellt werden. Es werden jedoch Kunstfehler toleriert, so dass ein falscher Alarm vermieden wird. Die Druckschrift offenbart also nur ein Alarmsystem, das auf lebensbedrohliche Situationen eines Patienten hinweist. Eine Vorrichtung zur qualitativen und/oder quantitativen Analyse von Schlafstörungen ist nicht offenbart.

Der Erfindung liegt die Aufgabe zugrunde, eine Vorrichtung zur qualitativen und/oder quantitativen Analyse von Schlafstörungen bereitzustellen, mit der im Schlaf auftretende Störungen genauestens und umfassend erfaßt werden, so daß neben der Analyse dieser Störungen auch daraus resultierende Störungen anderer Körperfunktionen erkannt werden können. Insbesondere soll mit der Vorrichtung die Erkennung von auf Schlafstörungen beruhenden Herzrhythmusstörungen (sog. "Apnoe-assoziierten Herzrhythmusstörungen") ermöglicht werden. Diese Aufgaben werden mit den Merkmalen der Ansprüche gelöst.

Bei der Lösung geht die Erfindung von folgenden Grundgedanken aus.

Um eine schnelle und genaue Analyse von Schlafstörungen und damit verbundenen Störungen durchführen zu können, ist es von großer Bedeutung, welche Körpersignale aufgenommen werden bzw. wieviele Parameter notwendig sind, um aus einer Korrelation oder einem Vergleich dieser Signale heraus auf die Ursache der Störungen schließen zu können. Aus diesem Grund besteht bei der vorliegenden Erfindung die Möglichkeit, das Herzpotential, den Blut-Sauerstoffgehalt, die Körperlage, Körperbewegungen, Atmungslaute, den Beatmungsdruck, den Atemfluß, die Atemtätigkeit und andere elektrophysiologische Meßwerte über verschiedene Sensoren zu messen und somit ein umfassendes Datenmaterial für die Analyse bereitzustellen. Die Meßsignale werden von den Sensoren an einen tragbaren Recorder geliefert, dort zwischengespeichert und nach dem Meßzyklus von einer Recheneinrichtung, wie etwa einem PC ausgelesen und verarbeitet. Der Recorder besitzt dazu eine Speicherkarte als externen Speicher. Diese wird für die Aufzeichnung der Signale verwendet und wird außerdem vor dem Meßbeginn für die anstehende Anwendung programmiert. So können durch die Auswahl eines entsprechenden Programmes gezielt bestimmte Sensoren angesprochen werden. Diese Zweiteilung der Hardwarekomponenten ermöglicht einen beliebigen stationären oder mobilen Einsatz der Vorrichtung. Zur Analyse der Meßdaten lassen sich diese zum einen direkt in unveränderter Form auf dem Bildschirm des PCs anzeigen, wobei natürlich beliebige Zeitbereiche ausgewählt werden können, zum anderen dienen die Daten jedoch auch als Grundlage für eine Auswertung, durch die weitere Kenngrößen ermittelt werden können. So wird z.B. das Herzpotentialsignal einem Herzfrequenzanalysator zugeführt, der daraus dircht oder über eine Korrelation mit anderen Meßwerten oder Histogrammdarstellungen die Veränderungen der Herzfrequenz ermittelt. Weiterhin werden die Meßsignale mittels geeigneter Rechenverfahren derart aufbereitet, daß z.B. eine Darstellung in Form von Tabellen oder Histogrammen erfolgen kann. Insbesondere die Sauerstoffsättigung des Blutes, die Positionswechsel des Patienten, Schnarchtätigkeiten und Atemstörungen lassen sich so übersichtlich und aussagekräftig darstellen.

Bei der Therapiekontrolle wird der Beatmungsdruck der Überdruckbeatmungsgeräte mitregistriert.

Mit einer Korrelation oder einem Vergleich dieser Daten können z.B. Schlafstörungen, Atmungsstörungen, Herzrhythmusstörungen erkannt werden. Insbesondere besteht der Vorteil, daraus Herzrhythmusstörungen zu erkennen, die auf Schlafstörungen beruhen. Gerade diese Art von Herzrhythmusstörungen läßt sich mit herkömmlichen Methoden wie z.B. Antiarythmika oder Herzschrittmachern nicht zufriedenstellend behandeln. Wenn jedoch ein ursächlicher Zusammenhang zwischen diesen Herzrhythmusstörungen und Schlafstörungen gezeigt werden kann, lassen sich die Herzrhythmusstörungen kausal durch eine Behandlung der Schlafstörungen beheben.

Um die Effektivität der Analyse zu erhöhen, werden außerdem Störsignale der Umgebung, sowie ein Absinken der Versorgungsspannung für die Auswertung berücksichtigt. Eine Überwachung der Versorgungsspannung verhindert einen Datenverlust, da bei einem Spannungsabfall die bis dahin gemessenen Daten gespeichert werden und für eine Auswertung zur Verfügung stehen. Die über die Sensorleitungen eingekoppelten Störsignale werden in verschiedene Frequenzbereiche gefiltert und können dann mit den Nutzsignalen verglichen werden, um so die auf Störfelder zurückgehenden Signalanteile in den Nutzsignalen zu lokalisieren. Diese Störanteile können somit bei der Analyse unberücksichtigt bleiben.

Weiterhin sind Alarmgeber vorgesehen, die beim Erreichen bestimmter Schwellwerte des Blutsauerstoffgehalts oder der Herzfrequenz auslösen und somit die Anwendung des Gerätes auch bei Risikopatienten zulassen.

Der Recorder ist vorteilhafterweise mit einer Echtzeituhr ausgestattet, so daß zum einen eine Echtzeitdarstellung der Signale, zum anderen ein Einsatz während eines ganz bestimmten Zeitraumes möglich ist. Dazu wird der Recorder in einen "Sleep"-Modus versetzt, und die Messungen beginnen erst zu einem gewünschten Zeitpunkt. Dies hat besondere Vorteile, wenn der Recorder in einem Institut voreingestellt und anschließend an den Patienten gesandt wird.

Als besondere Ausgestaltung besitzt die vorliegende Erfindung die Möglichkeit, auch EEG-Signale und Blutdruckmeßwerte anderer ambulanter Geräte aufzunehmen. Über das EEG-Signal läßt sich somit auch die Schlaftiefe erfassen.

Ein weiterer Vorteil der erfindungsgemäßen Vorrichtung kann darin gesehen werden, daß über einen Vergleich des Herzpotentialsignals mit dem Signal der Blut-Sauerstoffmessung die Pulswellen-Geschwindigkeit bzw. deren Veränderung bestimmt werden kann. Da diese mit den Schwankungen des Blutdrucks korreliert, ist zur Ermittlung dieser Schwankungen dann keine zusätzliche Blutdruckmessung mehr erforderlich.

Die erfindungsgemäße Vorrichtung ermöglicht nun ein Verfahren zur quantitativen Analyse von Schlafstörungen, bei dem ein mit einem Programm geladener Recorder von Meßsensoren aufgenommene Signale aufnimmt und speichert. Diese Daten werden danach (bei mobilem Einsatz) oder gleichzeitig (stationär) an eine Recheneinrichtung übertragen und von dieser verarbeitet. Die gemessenen und verarbeiteten Daten werden schließlich graphisch dargestellt.

Die Erfindung wird nachstehend mit Bezug auf die Zeichnungen näher erläutert. Es zeigen:

| | |
|---|---|
| Fig. 1a) und b) | graphische Darstellungen einer Auswahl der erfaßten Größen als Funktion der Zeit in zwei unterschiedlichen Zeitintervallen, |
| Fig. 2 bis 6 | Darstellungen einer Auswahl von Analyseergebnissen in Form von Tabellen (Fig. 2b, 3b, 3d, 4a, 4c) und Diagrammen (Fig. 3a, 3c, 4b, 5, 6) mit Patientendaten (Fig. 2a) |
| Fig. 7 | eine schematische Darstellung der erfindungsgemäßen Vorrichtung, |
| Fig. 8 | die Auswertung eines ersten Herzfrequenzsignals, |
| Fig. 9 | die Auswertung eines zweiten Herzfrequenzsignals, |
| Fig. 10 | die Auswertung eines respiratorischen Signals, |
| Fig. 11 | die Auswertung eines ersten Blut-Sauerstoffsättigungssignals vom Pulsoximeter, und |
| Fig. 12 | die Auswertung eines zweiten Blut-Sauerstoffsättigungssignals vom Pulsoximeter. |

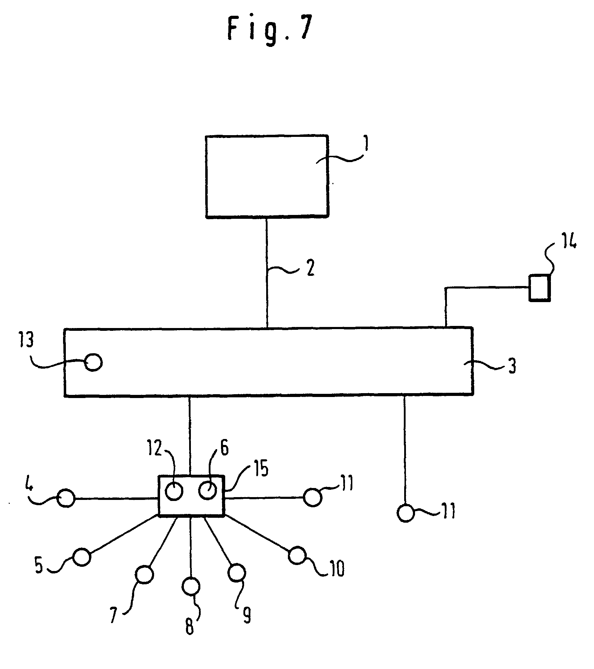

In folgenden wird zunächst näher auf die verwendeten Sensoren und die von diesen gelieferten Meßsignale eingegangen. Die erfindungsgemäße Vorrichtung ist dazu in Fig. 7 schematisch dargestellt.

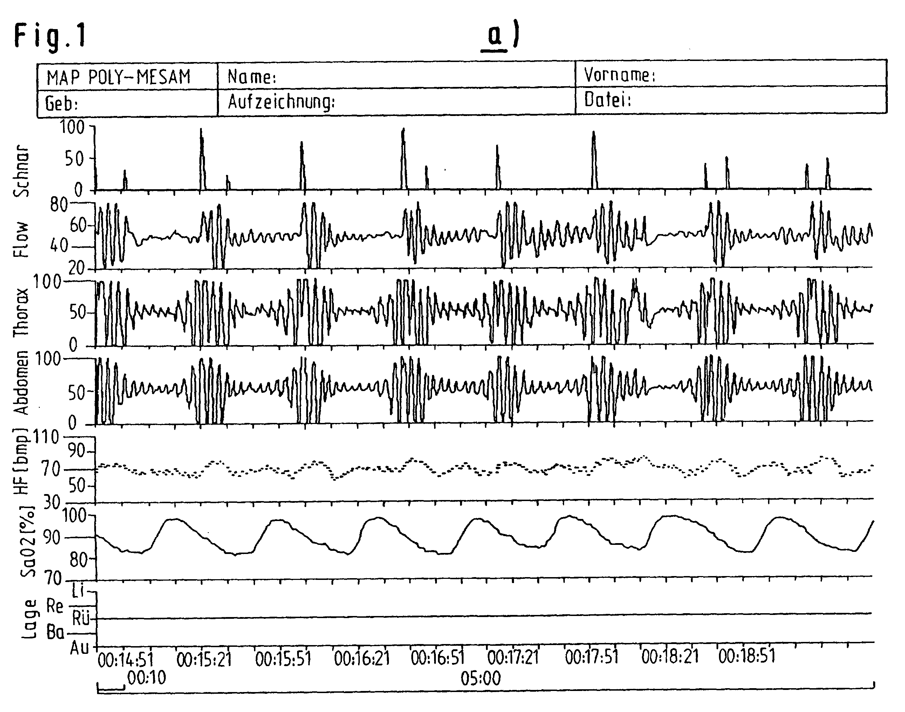

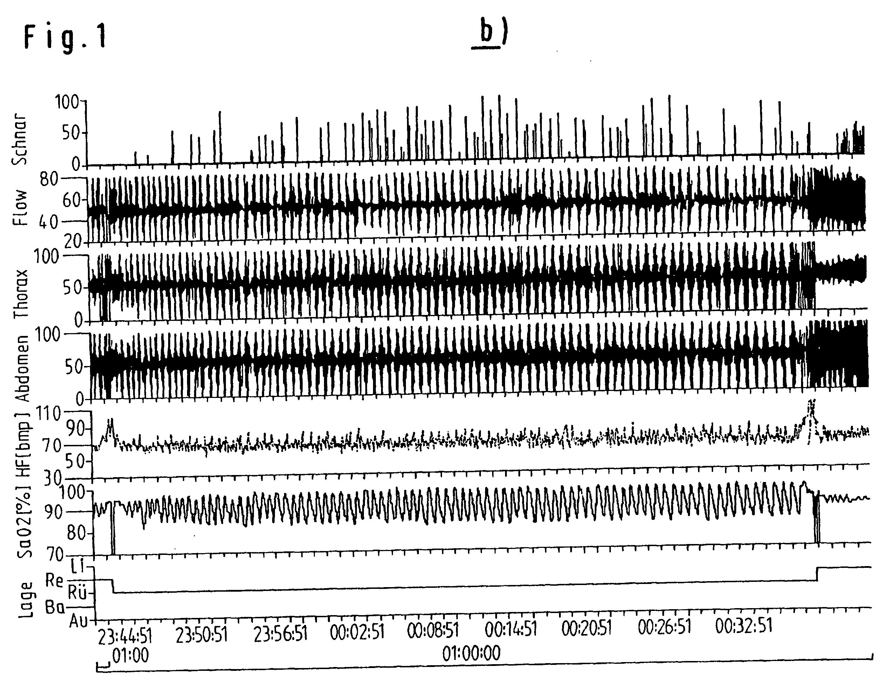

Die Atemgeräusche und Schnarchgeräusche des Patienten werden über ein am Larynx anzubringendes Elektretmikrophon 4 aufgenommen. Der Frequenzbereich dieses Mikrophons liegt dabei zwischen 50 bis 1500 Hz. Die von diesem Mikrophon gelieferte analoge Signalkurve wird rektifiziert und gefiltert, wonach die einhüllende Kurve mit einer über ein Konfigurationsmenü einstellbaren Frequenz abgetastet und digitalisiert wird. Der Atemfluß (Flow in Fig. 1) wird mittels eines Thermistors 5 oder eines Thermoelements gleichzeitig von Nase und Mund abgenommen. Dazu wird ein Summensignal gebildet, das zum einen die Atemfrequenzermittlung, zum anderen die Erkennung der Atemamplitude ermöglicht. Damit können zusammen mit den im folgenden beschriebenen Sensoren für Thorax und Abdomen auftretende Apnoen gut unterschieden werden. Die thorakale Atembewegung wird mit einem piezokeramischen Sensor 6 aufgenommen, der in ein Verteilerkästchen 15 eingebaut ist, an dem verschiedene Meßleitungen zusammenlaufen. Das Verteilerkästchen enthält Vorverstärker für die Meßaufnehmer. Für die abdominale Atembewegung wird ebenfalls ein piezokeramischer Sensor 7 eingesetzt. Zur Registrierung von Extremitätenbewegungen ist mindestens ein Aktographie-Sensor 8 vorgesehen. Dieser kann z.B. am Arm oder am Bein angebracht werden und bei der Verwendung eines zweiten Sensors, der über eine Y-Weiche mit dem ersten Sensor verbunden ist, lassen sich gleichzeitig Arm- und/oder Beinbewegungen feststellen. Mit diesen Aktographie-Sensoren lassen sich periodische Extremitätenbewegungen bestimmen, die sich im Vergleich zu Herzfrequenzvariationen zuordnen lassen. Zur Messung der Herzspannung wird auf das EKG-Verfahren zurückgegriffen. Dazu werden die drei Elektroden 9 an den standardisierten Stellen am Körper angebracht. Das analoge Herzpotential wird digitalisiert und als EKG-Signal gespeichert. Gleichzeitig wird das analoge EKG-Signal einer Spitzenwerterkennung zugeführt, um aus den zeitlichen Abständen zwischen den R-Zakken die Herzfrequenz bestimmen und speichern zu können. Die Messung der Sauerstoffsättigung des Blutes erfolgt mittels eines Pulsoximeters 10, dessen Sensor am Finger des Patienten angebracht wird. Ferner kann wie bei einer nasalen Überdrucktherapie über einen Drucksensor 11 der Beatmungsdruck direkt an der Maske während der Aufnahmezeit erfaßt werden. Der Drucksensor ist dabei mit einer Vorverstärkerelektronik und einer eigenen Stromversorgung in einem separaten Gehäuse eingebaut. Gemessen wird in einem Bereich von -10 mbar bis 30 mbar. Abschließend kann die Körperposition mittels eines Lagegebers 12, der z.B. aus vier Quecksilberschaltern besteht, ermittelt werden. Dieser Lagegeber zeigt stabil 5 Positionen an: rechts, links, Rückenlage, Bauchlage, aufrecht. Auch dieser Lagegeber kann in dem Verteilerkästchen integriert sein. Der Lagegeber ermöglicht die Bestimmung der tatsächlichen Zeit der Schlafposition und Zeiten unruhigen Schlafes, wodurch verschiedene Apnoephasen in Abhängigkeit von der Körperposition feststellbar sind, und auch Artefakte gut zugeordnet werden können. Ein besonderer Vorteil ist darin zu sehen, daß sowohl der Lagegeber als auch der Piezosensor für die thorakale Atembewegung in dem Verteilergehäuse integriert sind, wodurch die Anzahl der Meßpunkte am Körper des Patienten reduziert wird.

Im folgenden soll kurz der Ablauf einer Messung beschrieben werden.

Vor dem eigentlichen ambulanten oder stationären Betrieb wird die Vorrichtung über ein Ablaufprogramm auf der im Recorder 3 befindlichen Speicherkarte für die jeweilige Anwendung programmiert. Auf dieser Speicherkarte werden dann während der Aufzeichnung alle physiologischen Signale gespeichert. Die Programmierung des Recorders erfolgt durch das Einlesen eines Ablaufprogrammes für einen internen Mikroprozessor von der Speicherkarte, was sofort nach dem Einschieben der Speicherkarte geschieht. Der Recorder besitzt eine Echtzeituhr und kann auf einen bestimmten Startzeitpunkt bzw. Datum programmiert werden. Nach der Programmierung geht der Recorder in einen stromsparenden "Sleep-Modus" und wird von der Echtzeituhr zur programmierten Startuhrzeit und Datum geweckt und zu einer Stoppuhrzeit wieder abgeschaltet. Die Ablaufprogramme werden je nach medizinischer Fragestellung und der dafür notwendigen Konfiguration (Kanalzahl, Abtastraten, Startzeit, Stopzeit, Patientendaten usw.) von einem Computer 1 auf die Speicherkarte geschrieben. Es können je nach Anwendung Speicherkarten (PCMCIA-ATA-Standard) zwischen 1,8 MByte und 170 MByte Speicherkapazität benutzt werden. Die verschiedenen Speicherkapazitäten der Speicherkarten ermöglichen es somit, die physiologischen Signale mit verschiedenen Abtastraten und mit verschiedenen Aufnahmezeiten über die Ablaufprogramme zu konfigurieren. Die auf die Speicherkarte aufgezeichneten physiologischen Daten werden nach dem Einlesen in den Computer als Rohdaten ohne irgendeine Verfälschung gespeichert und sind zu jeder Zeit neu analysier- und darstellbar. Im stationären Echtzeitbetrieb wird der Recorder direkt mit dem Computer über eine serielle Schnittstelle 2 verbunden, die zur Übertragung der Daten während der Aufzeichnung an den PC dient.

Um bei dem ambulanten Einsatz des Recorders die Möglichkeit der fehlerhaften Diagnose durch Fehlfunktion oder unsachgemäßen Gebrauch durch den Patienten zu minimieren, ist es dem Arzt im Nachhinein möglich, den korrekten Ablauf der Aufzeichnung zu kontrollieren. Dazu wird z.B. die Versorgungsspannung aufgezeichnet, wobei ein Unterschreiten einer bestimmten Versorgungsspannung die Aufzeichnung abbricht. Die bereits aufgezeichneten Daten sind jedoch voll auswertbar.

Die Funktionalität des Recorders und der Sensoren wird nach der Applikation durch die Echtzeitdarstellung am Computer kontrolliert. Falls kein Computer zur Verfügung steht, wird die Kontrolle der Applikation anhand von LED-Leuchten, die auf der Frontplatte des Recorders angebracht sind, durchgeführt. Nach Einlesen des Ablaufprogrammes wird daraufhin ein 5-minütiger Testmodus gestartet, bei dem die LED-Leuchten bei Betätigung eines entsprechend zugeordneten Sensors aufleuchten. Dieses Testprogramm kann durch Betätigen einer Markertaste 13 erneut gestartet werden. Die Markertaste ist vorgesehen, um dem Patienten die Möglichkeit zu geben, bestimmte Ereignisse, wie z.B. ein Aufwachen während der Nacht, zu markieren.

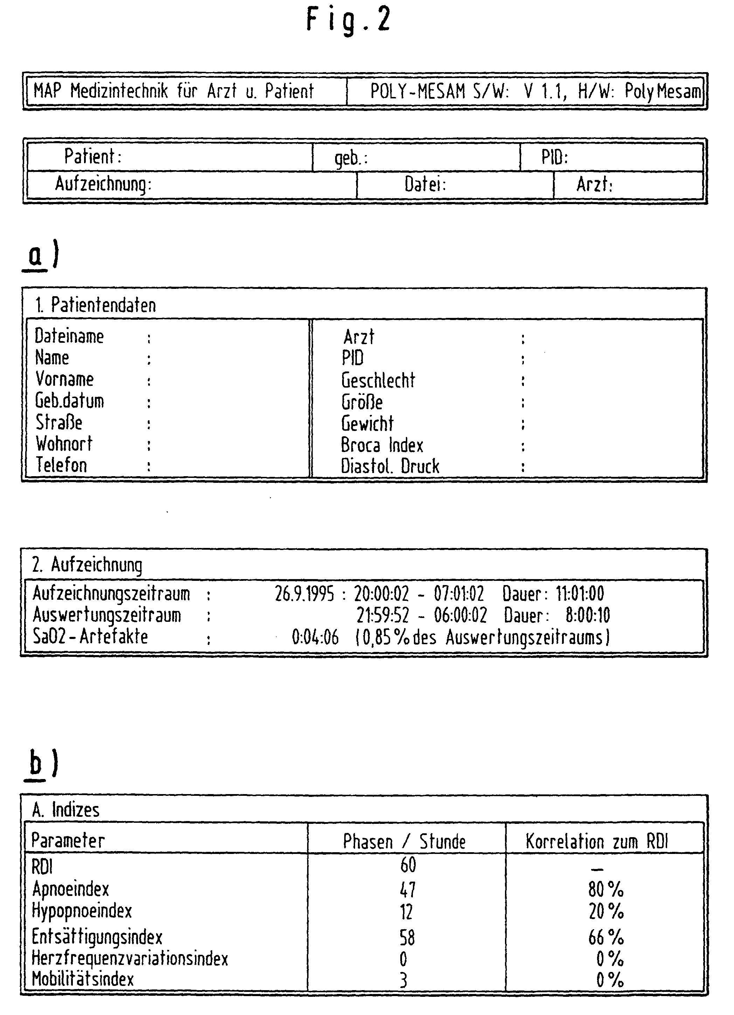

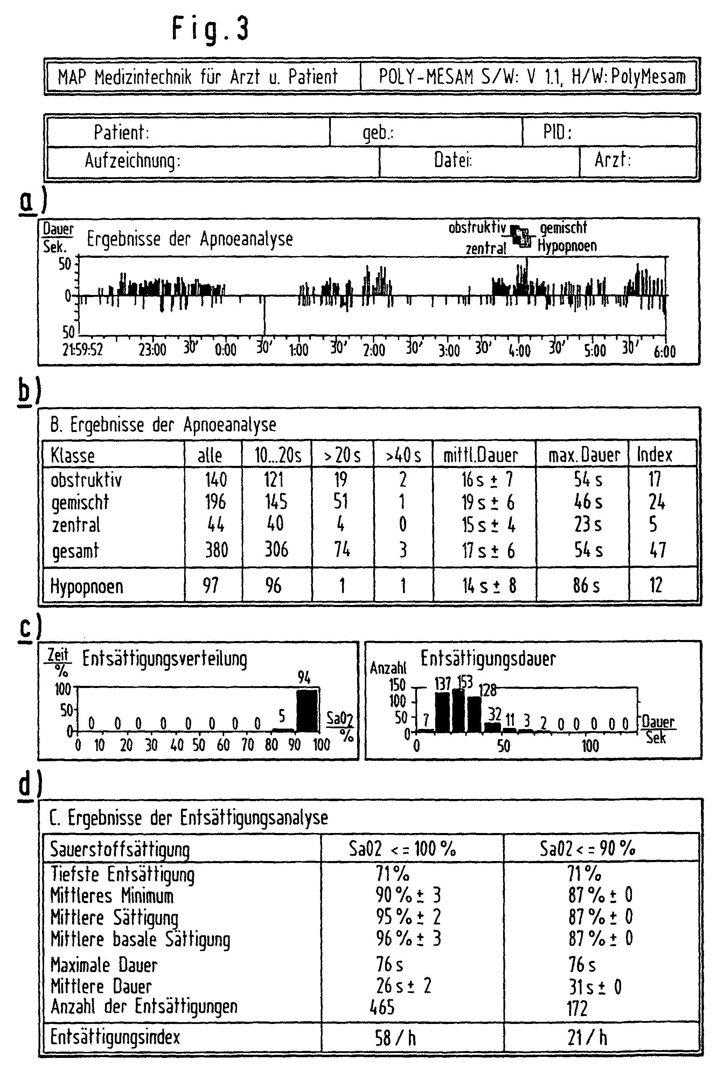

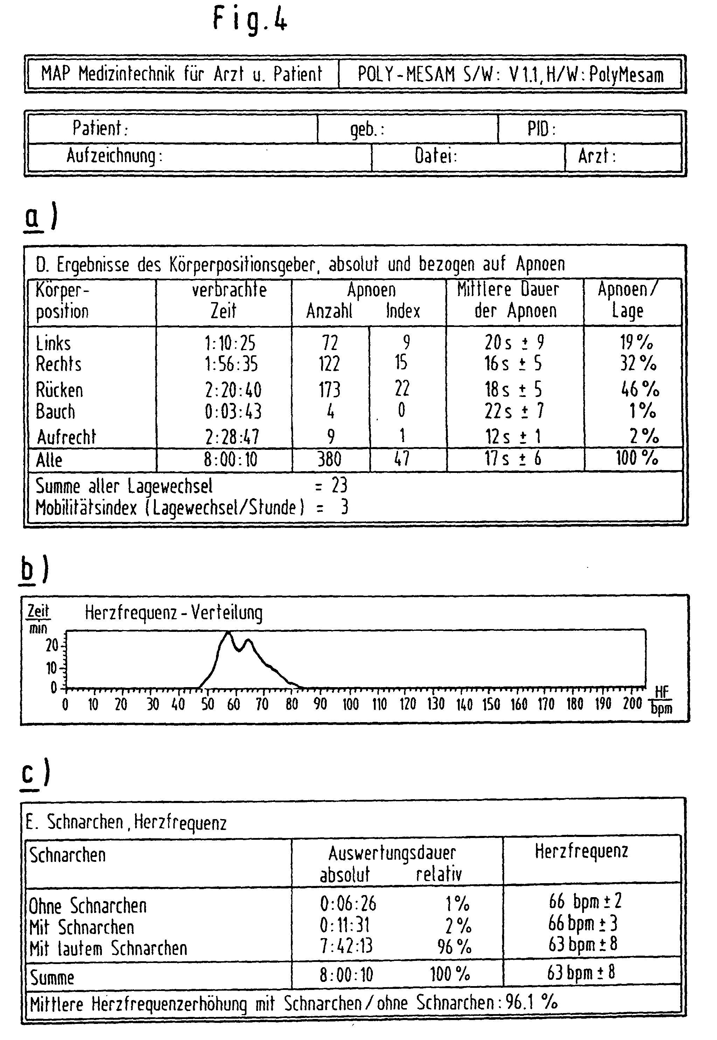

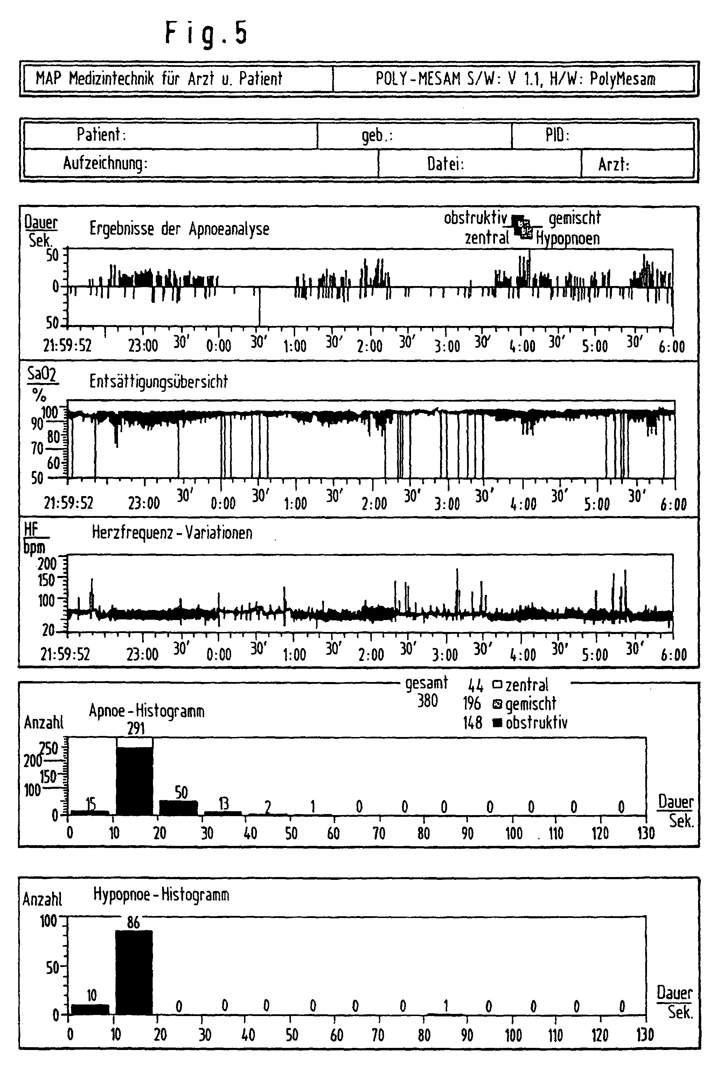

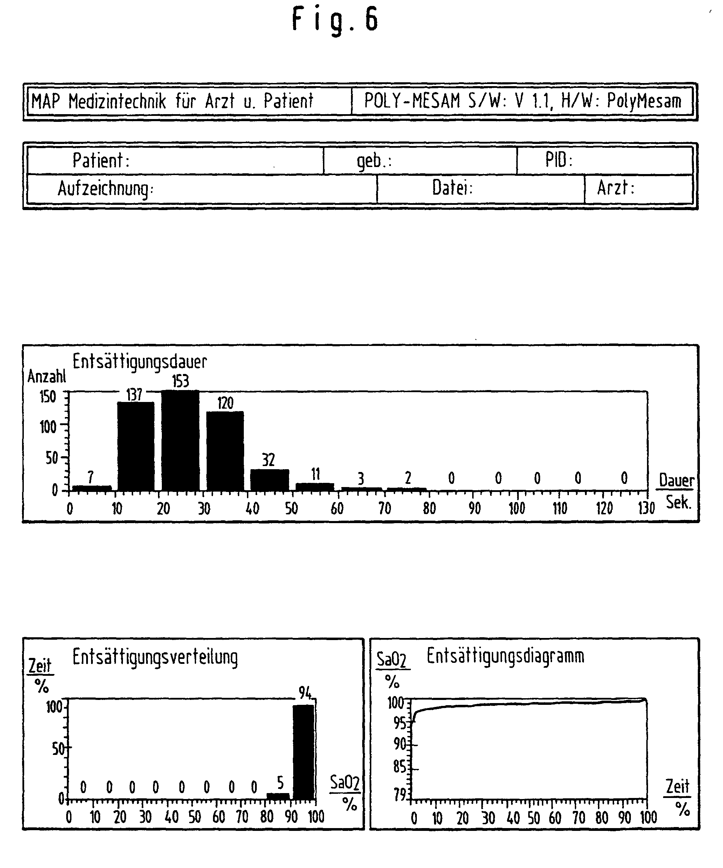

In den Figuren 2 bis 6 ist exemplarisch die Auswertung eines Meßzyklus abgebildet. Die Daten sind dabei auf verschiedene Weisen dargestellt. Signale, die bereits in der Rohfassung aussagekräftig sind, werden für einen gewünschten Zeitraum als Funktion der Zeit dargestellt. Die Daten der Blut-Sauerstoffmessung, der Lagesensoren, des Elektretmikrophons und der piezoelektrischen Sensoren zur Bestimmung der Atemtätigkeit werden nach statistischen Methoden ausgewertet und in Form von Tabellen bzw. Histogrammen veranschaulicht. Ebenfalls als Graph wird die Herzfrequenz-Verteilung (Fig. 4) dargestellt. Weiterhin ist, wie in Fig. 1 gezeigt, eine bloße zeitliche Abhängigkeit der Daten anzeigbar. Insbesondere die zeitlichen Verläufe der Meßsignale lassen sich beliebig kombiniert darstellen und eine kombinierte Anzeige von respiratorischen und kardiologischen Parametern ermöglicht es, bestimmte Herzrhythmusstörungen den aufgetretenen Schlafstörungen zuzuordnen. Diese Herzrhythmusstörungen werden am besten in der zeitlichen Darstellung der Herzfrequenz beobachtet und während der Auswertung qualitativ und quantitativ erfaßt. Die Herzfrequenzvariationen werden dabei automatisch bestimmt und daraus ein Herzfrequenzvariationsindex berechnet. Um die zyklischen Herzfrequenzvariationen, welche durch Schlafapnoen induziert werden, von denen durch Myoklonien induzierten Variationen unterscheiden zu können, wird das Herzfrequenzsignal mit dem Signal des Aktographie-Sensors verglichen. Die respiratorischen Ereignisse, d.h. die schlafbezogenen Atmungsstörungen werden während der Auswertung ebenfalls quantitativ analysiert und durch verschiedene daraus berechnete Indizes beschrieben. Diese sind wie in Fig. 2 unten zu sehen ist, ebenfalls in Form einer Tabelle angezeigt. Durch die Anwendung besonderer Auswertealgorithmen, die noch im folgenden beschrieben werden, ermöglicht es die vorliegende Erfindung zwischen Hypopnoen und obstruktiven, zentralen oder gemischten Apnoen zu unterscheiden. Weiterhin werden bei der automatischen Analyse der Apnoen und Hypopnoen die aufgezeichneten Druckschwankungen der nasalen Überdrucktherapie anstelle des Atemflußsignales herangezogen, wodurch eine exakte Therapiekontrolle ermöglicht wird.

Mit dem umfassenden Datenmaterial, das mit der vorliegenden Erfindung einem behandelnden Arzt zur Verfügung gestellt wird, wird diesem ermöglicht, eine exakte Diagnose bezüglich Schlafstörungen, Atmungsstörungen, und Herzrhythmusstörungen zu treffen. Der zu behandelnde Patient kann daraufhin gezielt therapiert werden.

Beispielhaft sind folgende Korrelationen denkbar, die im Rahmen der Erfindung automatisch ausgeführt werden können:

1. Eine Korrelation zwischen Schnarchgeräuschen und Sauerstoffreduktion gibt Hinweise auf obstruktive Apnoen.

2. Eine Korrelation zwischen rhythmischen Beinbewegungen (PLMs, Myoklonien) und Herzfrequenz, ohne Schnarchen und ohne Sauerstoffentsättigungen, ergeben Hinweise auf den Einfluß von Beinbewegungen auf das Herz/Kreislauf-System mit Arousal-Effekt.

3. Arrhythmien ohne Korrelationen zu einem anderen Signal deuten auf absolute Arrhythmie hin.

4. Arrhythmie synchron zu den Atemsignalen deutet auf Sinus-Arrhythmien und Apnoe-assoziierte Arrhythmien hin.

5. Kompensatorische Arrhythmie ohne Korrelation mit anderen Signalen deutet auf ventrikuläre Extrasystolen hin.

6. Regelmäßige Sauerstoff-Entsättigung mit synchroner Unterbrechung von Flow, sowie Thorax und Abdomenbewegung deutet auf zentrale Apnoen hin.

7. Langandauernde Sauerstoff-Entsättigungen mit evtl. unspezifischer Reduktion der Atmungsparameter und evtl. langsamer Änderung der Herzfrequenz, deuten auf eine obstruktive Lungenerkrankung hin.

8. Starke Apnoen mit starken synchronen Sauerstoff-Entsättigungen und geringen aber synchronen Herzfrequenzvariationen deuten auf Autonome Neuropathie hin (z.B. bei fortgeschrittenem Diabetes).

9. Absinken der Herzfrequenz nach dem Wechsel von der aufrechten in die horizontale Lage deuten auf Einschlafen hin. Bei gleichzeitigem Einsatz von Apnoen oder PLM ist der Einschlafzeitpunkt mit geringer Fehlertoleranz feststellbar.

10. Unregelmäßige Veränderungen, die etwa alle 1 1/2 Stunden gleichzeitig in allen Signalen auftauchen und ca. 20 - 40 Minuten andauern, deuten auf REM-Schlaf (Traumschlaf) hin.

11. Regelmäßiges Schnarchen ohne Sauerstoffentsättigung aber mit zyklischen Herzfrequenzerhöhungen weisen auf eine UARS (Upper Airways Resistence Syndrome) hin.

12. Als Maß für die Obstruktion wird die Differenz zwischen den Absolutwerten von Thorax- und Abdomenbewegung ermittelt und in einem Diagramm (z.B. in einem zusätzlichen Kanal) dargestellt.

Im folgenden wird auf die Auswertealgorithmen bezüglich der Herzfrequenzanalyse, der Algorithmus zur Erkennung von Apnoen und Hypopnoen und dem Algorithmus zur Erkennung von Sauerstoffentsättigungen eingegangen.
Ausgewertet werden die physiologischen Daten einerseits visuell anhand der Rohdaten und andererseits durch die automatisierten Auswerteprogramme auf dem Computer.

**Die automatische Herzfrequenzanalyse**

Algorithmus zur Erkennung von Herzfrequenzvariationen (Apnoeassoziierte Sinusarrhythmie)

Es wird hier mit dem R-Zackenabstand, dem sogenannten Tachogramm gearbeitet.
Der Algorithmus arbeitet die Tabelle mit den Herzfrequenz-Werten wie folgt ab. Die $h_i$ bezeichnen dabei die jeweiligen Herzfrequenzen, die $\Delta h_i$ die Differenzen aus den $h_i$.

Algorithmus zur Erkennung von Herzfrequenzanstiegen

n = maximale Dauer für Anstieg

- for i = 1, ..., n $\Delta h_i = h_i - h_{i-1}$
  Aufnehmen der $\Delta h_i$ in den Integrationspuffer. Der Integrationspuffer ist ein Vektor mit Dimension n, der mit Nullen initialisiert ist.

$$\text{Integrationspuffer} = \begin{pmatrix} \Delta h_1 \\ \cdot \\ \cdot \\ \Delta h_1 \\ 0 \\ \cdot \\ \cdot \\ 0 \end{pmatrix}$$

- Berechnen der Summe über die einzelnen Vektorelemente

$$\text{Integral} = \sum_{k=0}^{n} \Delta h_k$$

- Fallunterscheidung

  Integral <0 :
  Die Herzfrequenz-Kurve ist abgefallen. Der Integrationspuffer wird mit Nullen initialisiert.
  Integrationspuffer [i] = 0; i = 1, ..., n

  0 < Integral < Schwelle:
  Die Herzfrequenz-Kurve ist zwar angestiegen, aber nicht stark genug.

  Integral > Schwelle:

Die Herzfrequenz-Kurve ist um mindestens den Schwellenwert angestiegen. Das erste Kriterium für ein Ereignis ist erfüllt.

- Suche nach dem Maximum

Ab dem Beginn des Anstiegs wird nun nach dem Maximum gesucht. Dies wird durch Vergleichen der Herzfrequenz-Werte erreicht.

for n = 1, ..., Ende der Tabelle

eingelesener Wert > derzeitiges Maximum:
Wert = Maximum
Neuer Wert wird eingelesen

eingelesener Wert < derzeitiges Maximum:

**| Wert-Maximum | ≤ Abbruchschwelle:**
Die Herzfrequenz-Kurve ist wieder gefallen, aber nicht genug, als daß der Anstieg als beendet betrachtet werden könnte. Die Suche nach dem Maximum wird fortgesetzt.

**| Wert-Maximum | > Abbruchschwelle:**
Die Herzfrequenz-Kurve ist wieder abgefallen, und zwar mindestens um den Wert der Abbruchschwelle. Damit ist das Maximum gefunden. Von diesem gefundenen Maximum aus wird nun der Beginn und das Ende der Herzfrequenzvariation gesucht.

- Suche nach dem Beginn des Anstieges

Nun wird rückwärts in der Zeit ab dem gefundenen Maximum nach dem Beginn des Anstieges, dem 1. Minimum, gesucht. Die Suche erfolgt analog zur Maximumssuche. Die Tabellenwerte werden soweit rückwärts durchlaufen, bis das Ende des vorausgehenden Anstieges erreicht wird, maximal aber bis zum "Ende des Suchintervalles für das erste Minimum", ein Wert der durch den Benutzer vorgegeben werden kann.

for letztes Minimum, ..., Maximum:

eingelesener Wert < derzeitiges 1. Minimum:
Wert = 1. Minimum
Neuer Wert wird eingelesen.

eingelesener Wert > derzeitiges 1. Maximum:

**| Wert - 1. Minimum | ≤ Abbruchschwelle:**
Die Herzfrequenz-Kurve ist wieder angestiegen, aber nicht genug, als daß der Beginn des Anstieges als erreicht betrachtet werden könnte. Die Suche nach dem Beginn wird fortgesetzt.

**| Wert - 1. Minimum | > Abbruchschwelle:**
Die Herzfrequenz-Kurve ist wieder angestiegen, und zwar mindestens um den Wert der Abbruchschwelle. Damit ist der Beginn des Anstieges gefunden. Nun wird im nächsten Schritt das Ende des Anstieges bestimmt.

- Suche nach dem Ende des Herzfrequenzanstieges

Nun wird vorwärts in der Zeit ab dem gefundenen Maximum nach dem Ende des Anstieges, dem 2. Minimum, gesucht. Die Suche erfolgt analog zur Suche nach dem 1. Minimum. Die Tabellenwerte werden soweit vorwärts durchlaufen, bis ein Minimum gefunden wird, maximal aber bis zum "Ende des Suchintervalles für das zweite Minimum", ein Wert der durch den Benutzer vorgegeben werden kann.

for i = 1, ..., n :

eingelesener Wert < derzeitiges 2. Minimum:
Wert = 2. Minimum
Neuer Wert wird eingelesen.

eingelesener Wert > derzeitiges 2. Minimum:

**| Wert - 2. Minimum | ≤ Abbruchschwelle:**

Die Herzfrequenz-Kurve ist wieder angestiegen, aber nicht genug, als daß das Ende des Anstieges als erreicht betrachtet werden könnte. Die Suche nach dem Ende wird fortgesetzt.

**| Wert - 2. Minimum | > Abbruchschwelle:**

Die Herzfrequenz-Kurve ist wieder abgesunken, und zwar mindestens um den Wert der Abbruchschwelle. Damit ist das 2. Minimum gefunden.

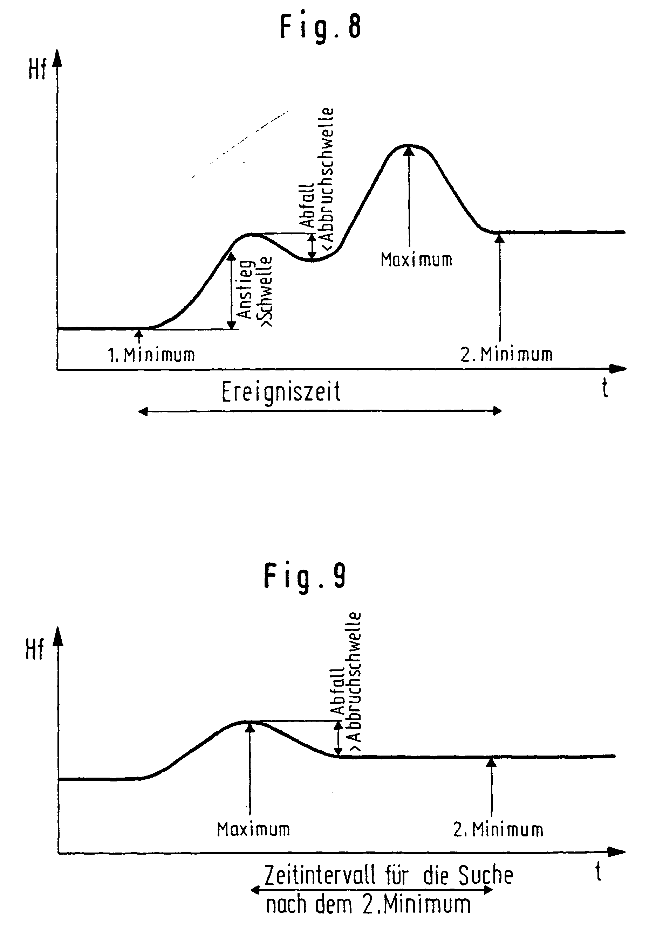

Analog zur $SaO_2$-Analyse spielen auch hier die einzelnen Parameter eine wichtige Rolle. Da das Herzfrequenzsignal wesentlich komplexer als das $SaO_2$-Signal ist, treten hier bestimmte Phänomene verschärft auf. In Fig. 8 ist beispielhaft die Herzfrequenz-Analyse dargestellt.

**Die Analyseparameter der Herzfrequenzanalyse**

Auch hier werden die einzelnen einstellbaren Parameter noch einmal genau erläutert.

- minimaler Herzfrequenz-Anstieg ∈ [1, 30]
  Dieser Wert legt fest, wie stark die Herzfrequenzkurve ansteigen muß, damit die Suche nach dem Maximum eingeleitet wird. Im Algorithmus ist dieser Wert die Integrationsschwelle.

- maximales Zeitintervall für Hf-Anstieg ∈ [1, 250]
  Dieser Wert beschränkt die Dauer, in der der Abfall der Hf-Kurve erfolgen muß. Im Algorithmus bedeutet er die Dimension des Integrations-Puffers.

- Abbruchschwelle für Min-Max-Suche ∈ [1, 10]
  Die Abbruchschwelle ist das Abbruchkriterium für eine Minimums- oder Maximumssuche bei einer Veränderung der Hf-Kurve um diesen Betrag.

- minimale und maximale Ereigniszeit ∈ [1, 250]
  Diese beiden Parameter sind letztendlich entscheidend dafür, ob ein gefundener Herzfrequenz-Anstieg als Ereignis in die Ereignisliste aufgenommen wird oder nicht. Um als Ereignis aufgenommen zu werden, muß für die Dauer des Anstieges gelten:
  min. Ereigniszeit $<(t_{2.Min} = t_{1.Min})$ < max. Ereigniszeit

- Suchintervall für das Maximum ∈ [1, 30]
  Es begrenzt die Suchumgebung für die Suche nach dem Maximum von einem aktuellen und damit zeitweiligen Maximum aus.

- Suchintervall für das erste Minimum ∈ [1, 30]
  Dieses Suchintervall begrenzt die Suchumgebung für die Suche nach dem Beginn des Herzfrequenzanstieges. Dieses Intervall ist ein zweites Abbruchkriterium nach der Abbruchschwelle. Es verhindert, daß sich der Algorithmus zu Tode läuft, wenn die Herzfrequenz in ein Plateau mündet (Fig. 8).

- Suchintervall für das zweite Minimum ∈ [1, 30]
  Dieses Suchintervall begrenzt die Suchumgebung für die Suche nach dem Ende des Anstieges. Dieses Intervall ist ein zweites Abbruchkriterium nach der Abbruchschwelle. Es verhindert, daß sich der Algorithmus zu Tode läuft, wenn die Herzfrequenzkurve in ein Plateau mündet (Fig. 9).

Die Grundeinstellung der Parameter ist:

- minimaler Herzfrequenz-Anstieg:　　　8
- Abbruchschwelle für Min-Max-Suche:　　　　8
- Suchintervall für das Maximum:　　　30 s
- Suchintervall für das erste Minimum:　　　　10 s
- Suchintervall für das zweite Minimum:　　　10 s
- minimale Ereigniszeit:　　　5 s
- maximale Ereigniszeit:　　　150 s

**Die respiratorischen Kanäle**

Algorithmus zur Erkennung von Apnoen und Hypopnoen

Der Algorithmus arbeitet in drei Schritten:

- Filterung der vorhandenen Daten
- Berechnung der Grenzwerte
- Durchführung der Analyse anhand der aufbereiteten Daten

Die Filterung der Daten ist notwendig, da das Signal der respiratorischen Kanäle kompliziert und störungsanfällig ist. Die Filterung erfolgt gleitend, immer über 1 Sekunde, das entspricht 250 Abtastwerten.

Filterung der Daten:

Die $f_i$ entsprechen den Abtastwerten des jeweiligen respiratorischen Kanals.

for i = 1, ..., 250

$$\Delta f_i = f_i - f_{i-1}$$

Aufnehmen der $\Delta f_i$ in den Differenzpuffer. Der Differenzpuffer ist ein Vektor mit Dimension 250, der mit Nullen initialisiert ist.

$$\text{Differenzpuffer} = \begin{pmatrix} \Delta f_1 \\ \cdot \\ \cdot \\ \Delta f_i \\ 0 \\ \cdot \\ \cdot \\ 0 \end{pmatrix}$$

Berechnung der Summen für den Integralpuffer

$$\text{sum}_k = \sum_{k=1}^{i} \Delta f_i$$

$$\text{Integralpuffer} = \begin{pmatrix} \text{sum}_1 \\ \cdot \\ \cdot \\ \text{sum}_k \\ 0 \\ \cdot \\ \cdot \\ 0 \end{pmatrix}$$

Mit diesen Werten wird nun weitergearbeitet.

Eine Apnoe oder Hypopnoe sind dadurch gekennzeichnet, daß die Atemkurve um einen gewissen Prozentsatz im Vergleich zur vorausgehenden Atmung absinkt. Da es sich bei der Atmung stets um eine sinuidale Kurve handelt und diese in seiner Amplitude auch bei gesunden Menschen schwankt, muß der Mittelwert über die vorausgehenden Atemzüge berechnet werden, damit ein Absinken der Atmung erkannt werden kann. Dies geschieht mit folgendem Algorithmus:

Algorithmus zur Bestimmung der Apnoe-/Hypopnoegrenzen

Der Mittelwert wird über die Minima und Maxima der letzten 10 Atemzüge berechnet.

Erkennung des Maximums:
$\text{sum}_i > 0$:

falls $\text{sum}_{i+1} > \max > 0$, d.h. die Atemkurve befindet sich noch im Ansteigen,
dann $\max = \text{sum}_{i+1}$
falls $\text{sum}_{i+1} < 0$, d.h.. die Kurve hat das Maximum längst überschritten,
dann ist das letzte berechnete Maximum gültig.

Erkennung des Minimums:
$\text{sum}_i < 0$:

falls $\text{sum}_{i+1} < \min < 0$, d.h. die Atemkurve befindet sich noch im Fallen,
dann $\min = \text{sum}_{i+1}$
falls $\text{sum}_{i+1} > 0$, d.h. die Kurve hat das Minimum längst überschritten,
dann ist das letzte berechnete Minimum gültig.

Über die Beträge der errechneten Minima und Maxima wird dann der Mittelwert gebildet:

$$\text{Mittel} = \frac{\sum_{i=1}^{5} |\min_i| + \max_i}{10}$$

Die Apnoe-/Hypopnoegrenzen berechnen sich dann aus

$$\text{Grenze} = \frac{\text{Ereignisschwelle} * \text{Mittel}}{100}$$

wobei die Ereignisschwelle in Prozent eingestellt werden kann.

Nun sind alle Hilfsmittel für die Analyse bereitgestellt.

Algorithmus zur Erkennung von Apnoen und Hypopnoen

for i = 1, ..., 250

falls | $sum_i$ | < Grenze,

setze Zähler = 0 und beginne zu zählen.

falls | $sum_i$ | > Grenze,

überprüfe, wie weit der Zähler gelaufen ist. Gilt: minimale Ereigniszeit < Zähler < maximale Ereigniszeit dann wurde eine Apnoe oder eine Hypopnoe gefunden.

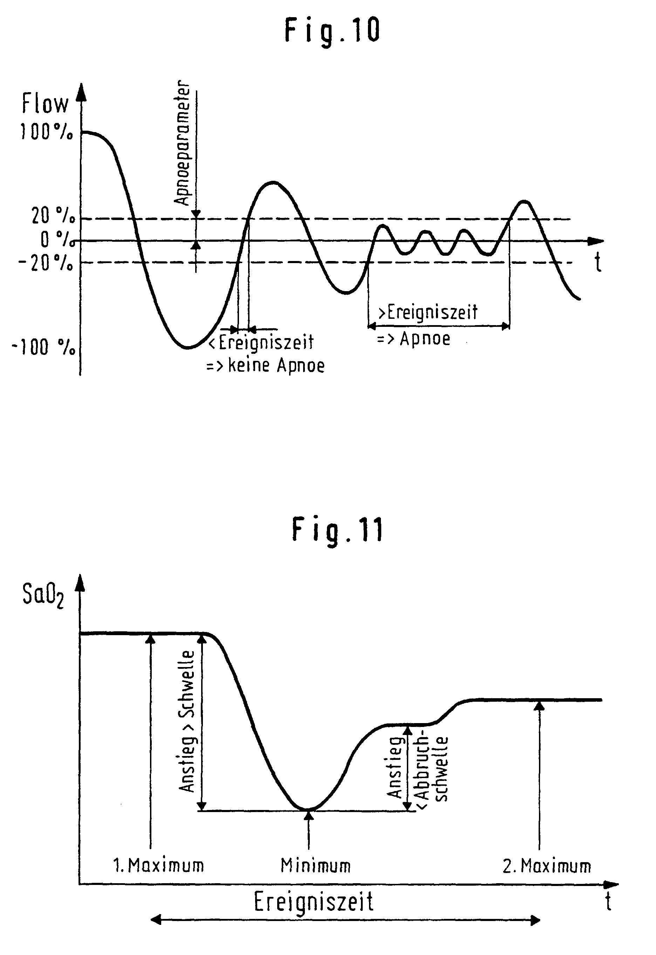

Diese Analyse wird auf allen respiratorischen Kanälen vollzogen. Obstruktive und zentrale Apnoe werden anhand der Thorax- und Abdomenaktivität differenziert. Sinkt die Atmung an Mund und Nase ab; überprüft das Programm, ob korreliert dazu die Aktivität auch an Thorax und Abdomen zurückgeht. Ist dies der Fall liegt eine zentrale Apnoe vor. Fig. 10 verdeutlicht die Analyse zur Erkennung von Apnoen.

**Die Analyseparameter der Apnoeanalyse**

Die Parameter bei der Apnoe-Analyse sind durch die medizinische Diagnostik festgelegt. Folgende Parameter sind einstellbar:

- Apnoe-Schwelle
  Damit eine Apnoe diagnostiziert werden kann, muß die Atemkurve um mindestens 80 % gegenüber dem Mittelwert über die letzten 10 Atemzüge absinken. Dieser Wert entspricht im Algorithmus der Ereignisschwelle

- minimale Apnoezeit
  Dieser Wert begrenzt das Zeitintervall, in der die Atemkurve unter der Apnoeschwelle liegen muß. Die minimale Apnoezeit muß mindestens 10 Sekunden betragen.

- Hypopnoe-Schwelle
  Damit eine Hypopnoe diagnostiziert werden kann, muß die Atemkurve um mindestens 50 % gegenüber dem Mittelwert über die letzten 10 Atemzüge absinken. Dieser Wert entspricht im Algorithmus der Ereignisschwelle.

- minimale Hypopnoezeit
  Dieser Wert begrenzt das Zeitintervall, in der die Atemkurve unter der Hypopnoeschwelle liegen muß. Die minimale Hypopnoezeit muß mindestens 10 Sekunden betragen.

- zentrale Apnoeschwelle
  Bei einer zentralen Apnoe muß die Amplitude der Thorax- und Abdomensignale um mindestens 80 % gegenüber dem Mittelwert über die letzten 10 Atemzüge absinken.

- minimale zentrale Zeit
  Dieser Wert begrenzt das Zeitintervall, in der die Thorax- und Abdomenkurven unter der zentralen Apnoeschwelle liegen müssen. Die minimale zentrale Apnoezeit muß mindestens 5 Sekunden betragen.

- maximale Dauer
  Dieser Wert beschränkt die maximale Dauer einer signifikanten Amplitudenreduktion für ein Ereignis.

**Der Sauerstoffsättigungskanal**

Algorithmus zur Erkennung von Entsättigungen

Ziel des Algorithmus ist die Sauerstoffentsättigungen zu erkennen. Da die Sauerstoffsättigung sehr hoch abgetastet wird, ist eine Datenreduktion nötig, damit die Analyse zügig erfolgen kann. Diese Datenreduktion geschieht, indem eine Tabelle erstellt wird, die jeweils den Mittelwert über eine Sekunde enthält. Dies bedeutet keine starke Einbuße an Information, da sich die Sauerstoffsättigung im Blut im Vergleich zur Herzfrequenz innerhalb einer Sekunde wenig ändert. Der Algorithmus durchsucht diese Tabelle dann nach signifikanten Ereignissen.

Dabei bedeuten die $S_i$ die jeweiligen $SaO_2$-Werte aus der Tabelle und die $\Delta S_i$ die Differenzen aus den $S_i$.

Algorithmus zur Erkennung von Sauerstoffentsättigungen

n = maximale Dauer für Abstieg

• for i = 1, ..., n

$$\Delta S_i = S_i - S_{i-1}$$

Aufnehmen der $\Delta S_i$, in den Tntegrationspuffer. Der Integrationspuffer ist ein Vektor mit Dimension n, der mit Nullen initialisiert ist.

$$\text{Integrationspuffer} = \begin{pmatrix} \Delta S_1 \\ . \\ . \\ \Delta S_i \\ 0 \\ . \\ . \\ 0 \end{pmatrix}$$

• Berechnen der Summe über die einzelnen Vektorelemente

$$\text{Integral} = \sum_{k=0}^{n} \Delta S_k$$

• Fallunterscheidung

Integral > 0:
Die $SaO_2$-Kurve ist angestiegen, d.h. keine Entsättigung ist indiziert. Der Integrationspuffer wird mit Nullen initialisiert.
Integrationspuffer [i] = 0; i = 1, ..., n

Schwelle > Integral > 0:
Die $SaO_2$-Kurve ist zwar abgefallen, aber nicht stark genug, als daß es sich um eine Entsättigung handeln könnte.

Integral < Schwelle:
Die $SaO_2$-Kurve ist um mindestens den Schwellenwert abgesunken. Das erste Kriterium für eine Entsättigung ist erfüllt.

• Suche nach dem Minimum
Ab dem Beginn des Absinkens wird nun nach dem Minimum gesucht. Dies wird durch Vergleichen der $SaO_2$-Werte erreicht.

EP 0 858 287 B1

for n = 1, ..., Ende der Tabelle

eingelesener Wert < derzeitiges Minimum:
Wert = Minimum
Neuer Wert wird eingelesen.

eingelesener Wert > derzeitiges Minimum:

**| Wert-Minimum | s Abbruchschwelle:**
Die SaO$_2$-Kurve ist wieder angestiegen; aber nicht genug,als daß die Entsättigung als beendet betrachtet werden könnte. Die Suche nach dem Minimum wird fortgesetzt.

**| Wert-Minimum | > Abbruchschwelle:**
Die SaO$_2$-Kurve ist wieder angestiegen, und zwar mindestens um den Wert der Abbruchschwelle. Damit ist das Minimum gefunden. Von diesem gefundenem Minimum aus wird nun der Beginn und das Ende der Entsättigung gesucht.

- Suche nach dem Beginn der Entsättigung
Nun wird rückwärts in der Zeit ab dem gefundenen Minimum nach dem Beginn der Entsättigung, dem 1. Maximum, gesucht. Die Suche erfolgt analog zur Minimumssuche. Die Tabellenwerte werden soweit rückwärts durchlaufen, bis das Ende der vorausgehenden Entsättigung erreicht wird, maximal aber bis zum "Ende des Suchintervalles für das erste Maximum", ein Wert der durch den Benutzer vorgegeben werden kann.
for letztes Maximum, ..., Minimum:

eingelesener Wert > derzeitiges 1. Maximum:
Wert = 1. Maximum
Neuer Wert wird eingelesen.

eingelesener Wert < derzeitiges 1. Maximum:

**| Wert - 1. Maximum | ≤ Abbruchschwelle:**
Die SaO$_2$-Kurve ist wieder abgesunken, aber nicht genug, als daß der Beginn der Entsättigung als erreicht betrachtet werden könnte. Die Suche nach dem Beginn wird fortgesetzt.

**| Wert - 1. Maximum | > Abbruchschwelle:**
Die SaO$_2$-Kurve ist wieder abgesunken, und zwar mindestens um den Wert der Abbruchschwelle. Damit ist das 1. Maximum gefunden, da jetzt die vorhergehende Entsättigung erreicht wurde. Nun wird im nächsten Schritt das Ende der Entsättigung bestimmt.

- Suche nach dem Ende der Entsättigung
Nun wird vorwärts in der Zeit ab dem gefundenen Minimum nach dem Ende der Entsättigung, dem 2. Maximum, gesucht. Die Suche erfolgt analog zur Suche nach dem 1. Maximum. Die Tabellenwerte werden soweit vorwärts durchlaufen, bis ein Maximum gefunden wird, maximal aber bis zum "Ende des Suchintervalles für das zweite Maximum", ein Wert der durch den Benutzer vorgegeben werden kann.
for i = 1, ..., n:

eingelesener Wert > derzeitiges 2. Maximum:
Wert = 2. Maximum
- Neuer Wert wird eingelesen.

eingelesener Wert < derzeitiges 2. Maximum:

**| Wert - 2. Maximum | ≤ Abbruchschwelle:**
Die SaO$_2$-Kurve ist wieder abgesunken, aber nicht genug, als daß das Ende der Entsättigung als erreicht betrachtet werden könnte. Die Suche nach dem Ende wird fortgesetzt.

**| Wert - 2. Maximum | > Abbruchschwelle:**
Die SaO$_2$-Kurve ist wieder abgesunken, und zwar mindestens um den Wert der Abbruchschwelle.

13

Damit ist das 2. Maximum gefunden, da jetzt die folgende Entsättigung erreicht wurde.

Dies war die Beschreibung des Grundalgorithmus. Eine wichtige Rolle für das effektive Funktionieren des Algorithmus spielt die richtige Wahl der Analyseparameter. Veranschaulicht wird die $SaO_2$-Analyse in Fig. 11.

Die Analyseparameter der $SaO_2$-Analyse

Wie bereits erwähnt, hängt die Effektivität des Algorithmus von der Wahl der geeigneten Parameter ab. Sämtliche Parameter sind frei einstellbar. Diese sollen im folgenden noch einmal detailliert beschrieben werden.

- minimaler $SaO_2$-Abfall $\in$ [-10, -1]
  Dieser Wert legt fest, um wieviel Prozent die Sauerstoffsättigungskurve abfallen muß, damit die Suche nach dem Minimum eingeleitet wird. Im Algorithmus ist dieser Wert die Integrationsschwelle.

- maximales Zeitintervall für $SaO_2$-Abfall $\in$ [1, 250]
  Dieser Wert beschränkt die Dauer, in der der Abfall der $SaO_2$-Kurve erfolgen muß. Im Algorithmus bedeutet er die Dimension des Integrations-Puffers.

- Abbruchschwelle für Min-Max-Suche $\in$ [1, 10]
  Die Abbruchschwelle ist das Abbruchkriterium für eine Minimums- oder Maximumssuche bei einer Veränderung der $SaO_2$-Kurve um diesen Betrag.

- minimale und maximale Ereigniszeit $\in$ [1, 250]
  Diese beiden Parameter sind letztendlich entscheidend dafür, ob eine gefundene Entsättigung als Ereignis in die Ereignisliste aufgenommen wird oder nicht. Um als Ereignis aufgenommen zu werden, muß für die Dauer der Sauerstoffentsättigung gelten min. Ereigniszeit < $(t_{2.Max} - t_{1.Max})$ < max Ereigniszeit

- Suchintervall für das Minimum $\in$ [1, 30]
  Es begrenzt die Suchumgebung für die Suche nach dem Minimum von einem aktuellen und damit zeitweiligen Minimum aus.

- Suchintervall für das erste Maximum $\in$ [1, 30]
  Dieses Suchintervall begrenzt die Suchumgebung für die Suche nach dem Beginn der Sauerstoffentsättigung. Dieses Intervall ist ein zweites Abbruchkriterium nach der Abbruchschwelle. Es verhindert, daß sich der Algorithmus zu Tode läuft, wenn die Sauerstoffsättigung in ein Plateau läuft.

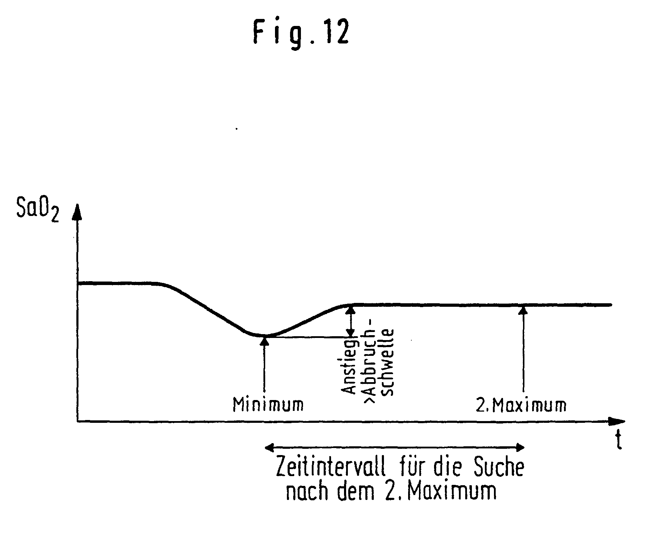

- Suchintervall für das zweite Maximum $\in$ [1, 30]
  Dieses Suchintervall begrenzt die Suchumgebung für die Suche nach dem Ende der Sauerstoffentsättigung. Dieses Intervall ist ein zweites Abbruchkriterium nach der Abbruchschwelle. Es verhindert, daß sich der Algorithmus zu Tode läuft, wenn die Sauerstoffsättigung in ein Plateau läuft (Fig. 12).

Die Grundeinstellung der Parameter ist:

- minimaler $SaO_2$-Abfall: -4 %
- Abbruchschwelle für Min-Max-Suche: 2 %
- Suchintervall für das Minimum: 30 s
- Suchintervall für das erste Maximum: 30 s
- Suchintervall für das zweite Maximum: 30 s
- minimale Ereigniszeit: 5 s
- maximale Ereigniszeit: 150 s

**Patentansprüche**

1. Vorrichtung zur qualitativen und/oder quantitativen Analyse von Schlafstörungen, mit mehreren Mitteln zur Erfassung von Körperfunktionen, einem mit den Mitteln verbundenen mobilen Recorder zur Aufnahme der Meßsignale der Mittel, und einer mit dem Recorder verbundenen Recheneinrichtung zur Speicherung und Auswertung der Meßsignale, mit

(a) einem Mittel (9) zum Erfassen des Herzpotentials,

(b) einem Pulsoximeter (10) zum Erfassen des Blut-Sauerstoffgehaltes,

(c) einer Einrichtung zum Ermitteln des Blutdrucks aus der Pulswellengeschwindigkeit und/oder deren Änderung, wobei die Pulswellengeschwindigkeit und/oder deren Änderung aus dem Herzpotentialsignal und dem Signal der Blutsauerstoffmessung ermittelt wird,

(d) einem Mittel (5) zum Erfassen des Atemflusses, wobei

(e) der Recorder eine Speicherkarte aufweist, die zur Aufzeichnung der Meßsignale verwendet wird, und

(f) der Recorder über die Speicherkarte vor Meßbeginn für die anstehende Anwendung zur Auswahllines Programms für die gezielte Ansprache bestimmter Sensoren der Mittel programmierbar ist.

2. Vorrichtung nach Anspruch 1, wobei das Mittel (9) zum Erfassen des Herzpotentials drei EKG-Elektroden aufweist.

3. Vorrichtung nach Anspruch 1 oder 2, wobei das Pulsoximeter (10) verschiedene Sensoren aufweist.

4. Vorrichtung nach einem der Ansprüche 1 bis 3, wobei mindestens ein weiteres Mittel aus der folgenden Gruppe vorgesehen ist:

(g) Mittel (4) zum Erfassen von Atmungslauten,

(h) Mittel (11) zum Erfassen des Beatmungsdrucks,

(i) Mittel (6, 7) zum Erfassen der Atemtätigkeit,

(j) Mittel (12) zum Erfassen der Körperlage,

(k) Mittel (8) zum Erfassen der Körperbewegungen, und

(l) Mittel zum Erfasssen elektrophysiologischer Parameter.

5. Vorrichtung nach Anspruch 4, wobei die Mittel (4) zum Erfassen von Atmungslauten ein Elektretmikrophon aufweisen.

6. Vorrichtung nach einem der Ansprüche 4 oder 5, wobei die Mittel zum Erfassen der Körperlage (12) vier Quecksilberschalter zum Erfassen von fünf Lagepositionen aufweisen.

7. Vorrichtung nach einem der Ansprüche 4 bis 6, wobei die Mittel (8) zum Erfassen der Körperbewegungen ein Piezoelement aufweisen.

8. Vorrichtung nach einem der Ansprüche 4 bis 7, wobei die Mittel (11) zum Erfassen des Beatmungsdrucks einen Drucksensor aufweisen.

9. Vorrichtung nach einem der Ansprüche 1 bis 8, wobei die Mittel (5) zum Erfassen des Atemflusses ein Thermoelement aufweisen.

10. Vorrichtung nach einem der Ansprüche 1 bis 9, wobei die Mittel (5) zum Erfassen des Atemflusses einen Thermistor aufweisen.

11. Vorrichtung nach einem der Ansprüche 4 bis 10, wobei die Mittel (6, 7) zum Erfassen der Atemtätigkeit ein Piezoelement aufweisen.

12. Vorrichtung nach einem der Ansprüche 4 bis 11, wobei die Mittel (6) zum Erfassen der Atemtätigkeit in die Mittel (12) zum Erfassen der Körperlage integriert sind.

13. Vorrichtung nach einem der Ansprüche 1 bis 12, wobei weiterhin Mittel (14) zum Aufnehmen externer ambulanter Signale vorgesehen sind.

14. Vorrichtung nach einem der Ansprüche 1 bis 13, wobei mindestens ein Alarmgeber vorgesehen ist.

15. Vorrichtung nach einem der Ansprüche 1 bis 14, wobei ein Alarmgeber den Blut-Sauerstoffgehalt überwacht.

16. Vorrichtung nach einem der Ansprüche 1 bis 15, wobei ein Alarmgeber die Herzfrequenz überwacht.

17. Vorrichtung nach einem der Ansprüche 1 bis 16, wobei Mittel zum Berücksichtigen von Störsignalen vorgesehen sind.

18. Vorrichtung nach einem der Ansprüche 1 bis 17, wobei die Vorrichtung für stationären oder mobilen Betrieb geeignet ist.

19. Vorrichtung nach einem der Ansprüche 1 bis 18 mit einer Einrichtung zum Korrelieren und/oder zum Vergleichen von respiratorischen und kardiologischen Signalen.

**Claims**

1. Apparatus for the qualitative and/or quantitative analysis of sleep disorders, comprising a plurality of means for detecting body functions, a mobile recorder connected to said means, for recording the measurement signals of the means, and a computer connected to the recorder for storing and evaluating the measurement signals, comprising

   (a) a means (9) for detecting the cardiac potential,
   (b) a pulse oximeter (10) for detecting the oxygen content of the blood,
   (c) a device for ascertaining the blood pressure from the pulse wave speed and/or a change therein, wherein the pulse wave speed and/or the change therein is ascertained from the cardiac potential signal and the signal from the measurement of the oxygen in the blood,
   (d) a means (5) for detecting the respiration flow, wherein
   (e) the recorder has a memory card which can be used for recording the measurement signals, and
   (f) the recorder is programmable by way of the memory card prior to the beginning of measurement for the impending use for the selection of a program for targetedly addressing given sensors of the means.

2. Apparatus according to claim 1 wherein the means (9) for detecting the cardiac potential has three ECG electrodes.

3. Apparatus according to claim 1 or claim 2 wherein the pulse oximeter (10) has various sensors.

4. Apparatus according to one of claims 1 to 3 wherein there is provided at least one further means from the following group:

   (g) means (4) for detecting respiratory sounds,
   (h) means (11) for detecting the artificial respiration pressure,
   (i) means (6, 7) for detecting the respiration activity,
   (j) means (12) for detecting the body position,
   (k) means (8) for detecting the body movements, and
   (l) means for detecting electrophysiological parameters.

5. Apparatus according to claim 4 wherein the means (4) for detecting respiratory sounds have an electret microphone.

6. Apparatus according to one of claims 4 and 5 wherein the means (12) for detecting the body position have four mercury switches for detecting five body positions.

7. Apparatus according to one of claims 4 to 6 wherein the means (8) for detecting the body movements have a piezoelectric element.

8. Apparatus according to one of claims 4 to 7 wherein the means (11) for detecting the respiration pressure have a pressure sensor.

9. Apparatus according to one of claims 1 to 8 wherein the means (5) for detecting the respiration flow have a thermoelement.

10. Apparatus according to one of claims 1 to 9 wherein the means (5) for detecting the respiration flow have a thermistor.

**11.** Apparatus according to one of claims 4 to 10 wherein the means (6, 7) for detecting the respiration activity have a piezoelectric element.

**12.** Apparatus according to one of claims 4 to 11 wherein the means (6, 7) for detecting the respiration activity are integrated into the means (12) for detecting the body position.

**13.** Apparatus according to one of claims 1 to 12 wherein there are further provided means (14) for recording external ambulant signals.

**14.** Apparatus according to one of claims 1 to 13 wherein there is provided at least one alarm device.

**15.** Apparatus according to one of claims 1 to 14 wherein an alarm device monitors the content of oxygen in the blood.

**16.** Apparatus according to one of claims 1 to 15 wherein an alarm device monitors the heart rate.

**17.** Apparatus according to one of claims 1 to 16 wherein there are provided means for taking account of interference signals.

**18.** Apparatus according to one of claims 1 to 17 wherein the apparatus is suitable for stationary or mobile operation.

**19.** Apparatus according to one of claims 1 to 18 comprising a device for correlating and/or comparing respiratory and cardiological signals.

**Revendications**

**1.** Dispositif pour l'analyse qualitative et/ou quantitative de troubles du sommeil comportant plusieurs moyens pour saisir des fonctions du corps, un enregistreur mobile relié avec les moyens pour enregistrer les signaux de mesure des moyens et un dispositif de calcul relié à l'enregistreur pour mémoriser et dépouiller les signaux de mesure, comportant

    (a) un moyen (9) pour saisir le potentiel cardiaque,
    (b) un pulsoximètre (10) pour saisir la teneur en oxygène du sang,
    (c) un dispositif pour déterminer la pression sanguine à partir de la vitesse des ondes du pouls et/ou de son changement, la vitesse des ondes du pouls et/ou son changement étant déterminés à partir du signal du potentiel cardiaque et du signal de la mesure de l'oxygène dans le sang,
    (d) un moyen (5) pour saisir le flux respiratoire,
    (e) l'enregistreur présentant une carte de mémoire qui est utilisée pour l'enregistrement des signaux de mesure et
    (f) l'enregistreur pouvant être programmé avant le début de la mesure pour l'application à exécuter moyennant la carte de mémoire pour le sélectionnement d'un programme qui active de manière ciblée des capteurs déterminés de moyens.

**2.** Dispositif selon la revendication 1, le moyen (9) pour saisir le potentiel cardiaque présentant trois électrodes ECG.

**3.** Dispositif selon les revendications 1 ou 2, le pulsoximètre (10) présentant divers capteurs.

**4.** Dispositif selon l'une quelconque des revendications 1 à 3, un moyen supplémentaire au moins parmi le groupe suivant étant prévu :

    (g) moyens (4) pour saisir les bruits respiratoires,
    (h) moyens (11) pour saisir la pression de la respiration assistée,
    (i) moyens (6, 7) pour saisir l'activité respiratoire,
    (j) moyens (12) pour saisir la position du corps,
    (k) moyens (8) pour saisir les mouvements du corps, et
    (l) moyens pour saisir les paramètres électrophysiologiques.

**5.** Dispositif selon la revendication 4, les moyens (4) pour saisir les bruits respiratoires présentant un microphone elektret.

6. Dispositif selon l'une quelconque des revendications 4 ou 5, les moyens pour saisir la position du corps (12) présentant quatre commutateurs au mercure pour saisir cinq positions du corps.

7. Dispositif selon l'une quelconque des revendications 4 à 6, les moyens (8) pour saisir les mouvements du corps présentant un élément piézoélectrique.

8. Dispositif selon l'une quelconque des revendications 4 à 7, les moyens (11) pour saisir la pression de la respiration assistée présentant un capteur de pression.

9. Dispositif selon l'une quelconque des revendications 1 à 8, les moyens (5) pour saisir le flux respiratoire présentant un thermoélément.

10. Dispositif selon l'une quelconque des revendications 1 à 9, les moyens (5) pour saisir le flux respiratoire présentant un thermistor.

11. Dispositif selon l'une quelconque des revendications 4 à 10, les moyens (6, 7) pour saisir l'activité respiratoire présentant un élément piézoélectrique.

12. Dispositif selon l'une quelconque des revendications 4 à 11, les moyens (6) pour saisir l'activité respiratoire étant intégrés dans les moyens (12) pour saisir la position du corps.

13. Dispositif selon l'une quelconque des revendications 1 à 12, des moyens supplémentaires (14) pour enregistrer des signaux ambulatoires externes étant prévus.

14. Dispositif selon l'une quelconque des revendications 1 à 13, au moins un émetteur d'alarme étant prévu.

15. Dispositif selon l'une quelconque des revendications 1 à 14, un émetteur d'alarme surveillant la teneur en oxygène du sang.

16. Dispositif selon l'une quelconque des revendications 1 à 15, un émetteur d'alarme surveillant la fréquence cardiaque.

17. Dispositif selon l'une quelconque des revendications 1 à 16, des moyens étant prévus pour tenir compte de signaux d'anomalie.

18. Dispositif selon l'une quelconque des revendications 1 à 17, le dispositif étant adapté au fonctionnement stationnaire ou mobile.

19. Dispositif selon l'une quelconque des revendications 1 à 18, comportant un dispositif pour mettre en corrélation et/ou pour comparer des signaux respiratoires et cardiologiques.

# Fig.1

<u>a</u>)

| MAP POLY-MESAM | Name: | Vorname: |
|----------------|-------|----------|
| Geb: | Aufzeichnung: | Datei: |

EP 0 858 287 B1

# Fig.1

## b)

EP 0 858 287 B1

# Fig.2

| MAP Medizintechnik für Arzt u. Patient | POLY-MESAM S/W:  V 1.1, H/W: PolyMesam |
|---|---|

| Patient: | geb.: | PID: |
|---|---|---|
| Aufzeichnung: | Datei: | Arzt: |

## a)

**1. Patientendaten**

| Dateiname | : | Arzt | : |
|---|---|---|---|
| Name | : | PID | : |
| Vorname | : | Geschlecht | : |
| Geb.datum | : | Größe | : |
| Straße | : | Gewicht | : |
| Wohnort | : | Broca Index | : |
| Telefon | : | Diastol. Druck | : |

**2. Aufzeichnung**

| Aufzeichnungszeitraum | : | 26.9.1995 : 20:00:02 – 07:01:02  Dauer: 11:01:00 |
|---|---|---|
| Auswertungszeitraum | : | 21:59:52 – 06:00:02  Dauer:  8:00:10 |
| SaO2–Artefakte | : | 0:04:06  (0,85 % des Auswertungszeitraums) |

## b)

**A. Indizes**

| Parameter | Phasen / Stunde | Korrelation zum RDI |
|---|---|---|
| RDI | 60 | — |
| Apnoeindex | 47 | 80 % |
| Hypopnoeindex | 12 | 20 % |
| Entsättigungsindex | 58 | 66 % |
| Herzfrequenzvariationsindex | 0 | 0 % |
| Mobilitätsindex | 3 | 0 % |

# Fig.3

| MAP Medizintechnik für Arzt u. Patient | POLY-MESAM S/W: V 1.1, H/W: PolyMesam |
|---|---|

| Patient: | geb.: | PID: |
|---|---|---|
| Aufzeichnung: | Datei: | Arzt: |

## a)

Dauer/Sek. — Ergebnisse der Apnoeanalyse — obstruktiv, zentral, gemischt, Hypopnoen

## b)

### B. Ergebnisse der Apnoeanalyse

| Klasse | alle | 10...20s | >20 s | >40 s | mittl.Dauer | max.Dauer | Index |
|---|---|---|---|---|---|---|---|
| obstruktiv | 140 | 121 | 19 | 2 | 16 s ± 7 | 54 s | 17 |
| gemischt | 196 | 145 | 51 | 1 | 19 s ± 6 | 46 s | 24 |
| zentral | 44 | 40 | 4 | 0 | 15 s ± 4 | 23 s | 5 |
| gesamt | 380 | 306 | 74 | 3 | 17 s ± 6 | 54 s | 47 |
| Hypopnoen | 97 | 96 | 1 | 1 | 14 s ± 8 | 86 s | 12 |

## c)

Zeit/% — Entsättigungsverteilung — SaO2/%

Anzahl — Entsättigungsdauer — Dauer/Sek

## d)

### C. Ergebnisse der Entsättigungsanalyse

| Sauerstoffsättigung | SaO2 <= 100 % | SaO2 <= 90 % |
|---|---|---|
| Tiefste Entsättigung | 71 % | 71 % |
| Mittleres Minimum | 90 % ± 3 | 87 % ± 0 |
| Mittlere Sättigung | 95 % ± 2 | 87 % ± 0 |
| Mittlere basale Sättigung | 96 % ± 3 | 87 % ± 0 |
| Maximale Dauer | 76 s | 76 s |
| Mittlere Dauer | 26 s ± 2 | 31 s ± 0 |
| Anzahl der Entsättigungen | 465 | 172 |
| Entsättigungsindex | 58 / h | 21 / h |

# Fig.4

| MAP Medizintechnik für Arzt u. Patient | POLY-MESAM S/W: V 1.1, H/W: PolyMesam |
|---|---|

| Patient: | | geb.: | PID: |
|---|---|---|---|
| Aufzeichnung: | | Datei: | Arzt: |

## a )

D. Ergebnisse des Körperpositionsgeber, absolut und bezogen auf Apnoen

| Körper-position | verbrachte Zeit | Apnoen | | Mittlere Dauer der Apnoen | Apnoen/ Lage |
|---|---|---|---|---|---|
| | | Anzahl | Index | | |
| Links | 1:10:25 | 72 | 9 | 20 s ± 9 | 19 % |
| Rechts | 1:56:35 | 122 | 15 | 16 s ± 5 | 32 % |
| Rücken | 2:20:40 | 173 | 22 | 18 s ± 5 | 46 % |
| Bauch | 0:03:43 | 4 | 0 | 22 s ± 7 | 1 % |
| Aufrecht | 2:28:47 | 9 | 1 | 12 s ± 1 | 2 % |
| Alle | 8:00:10 | 380 | 47 | 17 s ± 6 | 100 % |

Summe aller Lagewechsel = 23
Mobilitätsindex (Lagewechsel/Stunde) = 3

## b )

Zeit/min — Herzfrequenz - Verteilung

## c )

E. Schnarchen, Herzfrequenz

| Schnarchen | Auswertungsdauer | | Herzfrequenz |
|---|---|---|---|
| | absolut | relativ | |
| Ohne Schnarchen | 0:06:26 | 1 % | 66 bpm ± 2 |
| Mit Schnarchen | 0:11:31 | 2 % | 66 bpm ± 3 |
| Mit lautem Schnarchen | 7:42:13 | 96 % | 63 bpm ± 8 |
| Summe | 8:00:10 | 100 % | 63 bpm ± 8 |

Mittlere Herzfrequenzerhöhung mit Schnarchen / ohne Schnarchen : 96.1 %

# Fig.5

| MAP Medizintechnik für Arzt u. Patient | POLY-MESAM S/W: V 1.1, H/W: PolyMesam |
|---|---|

| Patient: | geb.: | PID: |
|---|---|---|
| Aufzeichnung: | Datei: | Arzt: |

**Dauer/Sek.** — Ergebnisse der Apnoeanalyse — obstruktiv ■, gemischt, zentral, Hypopnoen

**SaO2/%** — Entsättigungsübersicht

**HF/bpm** — Herzfrequenz-Variationen

gesamt 44 zentral
380 196 gemischt
148 obstruktiv

Anzahl — Apnoe-Histogramm
291
15, 250, 50, 13, 2, 1, 0, 0, 0, 0, 0, 0, 0

Anzahl — Hypopnoe-Histogramm
86
10, 0, 0, 0, 0, 0, 0, 1, 0, 0, 0, 0

# Fig. 6

| MAP Medizintechnik für Arzt u. Patient | POLY-MESAM S/W: V 1.1, H/W: PolyMesam |
|---|---|

| Patient: | geb.: | PID: |
|---|---|---|
| Aufzeichnung: | Datei: | Arzt: |

Entsättigungsdauer

Entsättigungsverteilung

Entsättigungsdiagramm

# Fig.7

# Fig. 8

# Fig. 9

# Fig.10

# Fig.11

# Fig.12

$SaO_2$

Minimum

Anstieg
>Abbruch-
schwelle

2.Maximum

t

Zeitintervall für die Suche
nach dem 2.Maximum